# EUROPEAN PATENT APPLICATION

(11) **EP 4 386 774 A1**
(43) Date of publication of application: **19.06.2024**
(21) Application number: 22214042.8
(22) Date of filing: 16.12.2022
(51) Int. Cl.: G16H 40/67, G16H 40/40

(54) **WAITING ROOM TRIGGERED INTERROGATION ON DEMAND (IOD)**

(71) Applicant: BIOTRONIK SE & Co. KG, 12359 Berlin (DE)
(72) Inventor: Krüger, Daniel, 15741 Bestensee (DE); Deutschmann, Hendrik, 10247 Berlin (DE); Müller, Jens, 14195 Berlin (DE)
(74) Representative: Biotronik Corporate Services SE

(57) **Abstract**

A remote server for providing medical device data of a medical device of a patient may be provided, wherein the remote server may be configured to receive (i) location data on a scheduled appointment of the patient, (ii) time data on a scheduled appointment of the patient, (iii) emergency data on at least one emergency event associated with the patient, and/or (iv) trigger data providing an instruction to the remote server. The remote server may further be configured to send, at least in part based on the received data, an interrogation request to the medical device.

## Description

The present invention relates to a remote server, a remote device, and an according system for providing medical device data of a medical device of a patient as well as respective computer programs and methods.

When a patient with a medical implant arrives for a doctor's appointment or, e.g., in case of an emergency, in a hospital, the medical staff needs to interrogate the patient's implant with a programming instance to get the actual implant dataset, e.g., the medical device data. This is a time-consuming process, which may even delay the treatment for such long times that treatment success chances may decrease.

The medical staff may not know, if a patient wears an (implanted) medical device, and even if they do, sending an interrogation request, e.g., at the beginning of the appointment, may result in a time-consuming interrogation to retrieve the medical device data, wasting valuable time during the appointment. This reduces the overall workflow efficiency, affecting the health professionals, the patient and further patients waiting for upcoming appointments.

Also, when the medical staff fails to identify a medical device providing relevant information on the patient's health state, the administered treatment might be not ideal or, in the worst case, even harmful as the assessment may not be based on all potentially available information on the patient. Depending on the patient's condition, he/she might potentially not be able to correctly communicate relevant medical device-related information to the medical staff in preparation for medical device interrogation.

More generally, issues may arise when the information collected by a medical device is not available to the respective health professional at all or not on time.

Therefore, there is a need to optimize the provision of medical device data of a medical device of a patient.

This need is met at least in part by the various aspects of the invention outlined herein.

According to one aspect of the invention, a remote server for providing medical device data of a medical device of a patient is provided. The remote server is configured to receive at least one element of the following list of elements: (i) location data on a scheduled appointment of the patient, (ii) time data on a scheduled appointment of the patient, (iii) emergency data on at least one emergency event associated with the patient, and/or (iv) an interrogation instruction. The remote server is further configured to send, at least in part based on the at least one element, an interrogation request to the medical device.

Generally, this allows to interrogate a medical device to retrieve medical device data on demand, based at least in part on the elements (i) through (iv), which improves the workflow in a typical appointment of a patient carrying a medical device. It allows for data-driven automatization of such workflow, minimizing the need for human intervention and thus saving valuable time for the patient, the health professionals, and further patients waiting for their subsequent appointments with that health professional(s). This may increase success chances of the respective treatment during the appointment, e.g., in case of emergencies. The remote server may comprise one or more (remote) servers and/or one or more cloud-based servers, for example.

For example, an interrogation request may be (automatically) sent, if the patient enters the location of an appointment and/or the time of an appointment of the patient is imminent. Thus, the interrogation request may be sent early, or just-in-time, such that, the medical device data is available to the treating doctor at the right time, without causing delays.

Additionally or alternatively, the interrogation request may be sent from the remote server side in case of an emergency and/or upon receiving an interrogation instruction.

The location and/or time data may comprise the location(s) and/or time(s), respectively, of one or more scheduled appointments of the patient with the same or different health professionals. Also, the time data may comprise the times of other scheduled appointments at which the medical device data may be requested, e.g., for a regular remote check-up, even if the scheduled appointment does not involve the patient visiting a health institution.

Time data and location data may be provided to the remote server as vectors, each comprising both the time and location of an appointment. Location data on a scheduled appointment of the patient, e.g., on predetermined areas associated with the locations of, e.g., health institutions may also be received separately by the remote server. Similarly, location data on a current location of the patient may be received. Analogously, time data on the current time and/or time data on a scheduled appointment of the patient may also be received separately by the remote server. The time data may be provided by the concerned health professional(s) or the patient via, e.g., a calendar entry comprising a date and a time, and, optionally, a location (such as to also convey location data as discussed above). The time and/or location data may thus be received from a patient's device and/or from a doctor's device or a hospital device.

The location data may, e.g., comprise the locations of hospitals, doctor's offices and other healthcare institutions, for example in form of predetermined areas around them. These location data may be stored in the remote server and/or received by the remote server. The location data may define a predetermined area for each health institution, for example the actual area of the building, or a circular area around a defined position associated with the health institution. The location data may for example be provided by health professionals, the patient and/or other patients, an online database (e.g., online maps services), etc. The location data may be provided by health professionals or patients via, e.g., a calendar entry comprising an address. In other examples, the remote server may retrieve the location based on a calendar entry comprising a name (e.g. hospital name).

The emergency data may comprise information on emergency events associated with the patient. The emergency events may, for example, be detected by the medical device and/or a remote device (e.g. a patient's device). The emergency events may be related to a health state of the patient. For example, a mobile phone as a remote device (but also a medical implant) may detect that the patient has fallen, the medical device, e.g., an electrocardiograph, may detect an unexpected change in any parameter associated with the cardiac system of the patient, e.g., heart rhythm, heartrate, etc.

In an example, a remote device may receive the time data and the location data and may be configured to process them and send, at least in part based thereon, an interrogation instruction to the remote server. In this exemplary embodiment, the remote server may not require the location data and time data but may send an interrogation request to the medical device simply when receiving the interrogation instruction.

The interrogation request may instruct the medical device to acquire medical device data associated with a health state of the patient and to send the medical device data to the remote server. The interrogation request may comprise instructions for the medical device to determine the health state of the patient according to at least one parameter. When the medical device receives the interrogation request, it may perform an interrogation according to the at least one parameter of the interrogation request and/or a predetermined interrogation protocol to acquire medical device data. This interrogation may comprise one or more measurements of associated parameters which may then be stored as the medical device data. These may then be sent to the remote server and from there to a patient data system. In other examples, the medical device may simply be adapted to trigger that data stored by the medical device be sent to the remote server. The medical device may provide the medical device data to the remote server in the form as they were acquired or in a processed form. Optionally, the medical device may, e.g., process the medical device data before sending the medical device data to the patient data system.

The medical device may for example be an electrocardiograph, a cardiac valve implant, a pacemaker, an implantable cardioverter defibrillator (ICD), a cochlear implant, brain pacemaker, a neurostimulation device, etc. The medical device may comprise an interface for wired or wireless communication with the remote server and/or one or more remote devices. Any functionality described with reference to the medical device, the remote server and/or the remote device may also be fully or partly be implemented in any of the other components.

For example, the remote server may cooperate with one or more remote devices, which may or may not be in accordance with a further aspect of the present invention as described in more detail below. A remote device may for example be a mobile phone, in particular a smartphone, a smart watch, a tablet, a computer, etc. In some examples, one or more functions of the remote server may be outsourced to the remote device, e.g. an application running on the remote device.

Multiple remote devices and/or multiple medical devices may cooperate and send and/or receive time data, location data, and/or interrogation instructions to/from one or more remote servers.

In an exemplary embodiment, the remote server may be configured to send the interrogation request to the medical device, if at least one of the following events occurs: (i) a current location of the patient is within a predetermined area of a location of the scheduled appointment, (ii) a current time is within a predetermined range around the time of the scheduled appointment, and (iii) emergency data on at least one emergency event associated with the patient is received, and (iv) the interrogation instruction is received.

Firstly, the interrogation request may be sent to the medical device when the remote server receives location data indicating that the current location is within a predetermined area (potentially out of a plurality of predetermined areas, e.g. association with different appointments). The predetermined area may for example be the location of a health institution. The predetermined area associated with the location of a health institution may for example be defined by the area within a radius of, e.g., 10 m, 20 m, 30 m, 40 m, 50 m, 60 m, 70 m, 80 m, 90 m, 100 m, 200 m, 300 m, 400 m, 500 m, 1 km, or more around a location, e.g., at or near the center of the respective health institution, defined by longitude and latitude coordinates. The predetermined area may be provided by the patient data system, e.g., via a doctor's PC. Upon arrival of the patient, the current location of the patient, measured for example by the remote device of the patient (e.g., a mobile phone) is within the predetermined area associated with a hospital or doctor's office.

A current location of the patient may for example be provided via a mobile phone, a smart watch, and/or any other suitable (remote) device, for instance any (remote) device with a locating (e.g., GPS) function. The current location may be updated (quasi-)continuously and/or at a predetermined frequency, e.g., every minute or every second.

Secondly, the interrogation request may be sent to the medical device when the current time is within a predetermined range around the time (or: before the time) of a scheduled appointment. The predetermined range may for example be 5 minutes, 10 minutes, 15 minutes, 20 minutes, 25 minutes, 30 minutes, 1 hour or any other time before the start of the appointment. The current time may be provided for example by a remote device (suitably equipped with a clock) or by an online service or by the remote server itself.

Thirdly, the interrogation request may be sent to the medical device when the remote server receives emergency data indicating at least one emergency event of the patient. The remote server may additionally, when an emergency event is indicated, send a warning message to a server running a patient data system, e.g., of a health professional.

Fourthly, the remote server may receive the interrogation instruction e.g., from a remote device. The remote device may for example be a mobile phone. In an exemplary embodiment, the remote device may be configured to automatically compare the current time and location with the time and location of a scheduled appointment of the patient and send an interrogation instruction to the remote server, when both the current time and the current location of the patient match time and location of that scheduled appointment. Further details on the remote device and on alternative embodiments thereof are provided below.

Sending the interrogation request based on various kinds of events makes the remote server suitable to respond in all necessary situations and thus increases patient comfort, satisfaction, and safety.

Multiple of the above events may occur at the same time. For example, when the patient has a scheduled appointment, typically the patient is at the location of the appointment (e.g. health institution) at a time within a predetermined range around the time of the scheduled appointment. Additionally, the medical device may optionally have sent emergency data indicating an emergency event associated with the patient, potentially related to the reason why the patient is seeing a health professional. The system may be configured such that, in cases like this, the medical device data are only sent once to the respective patient data system to be available for the health professional and/or the data are only stored in the respective electronic patient file once. For example, the interrogation request may be sent only in case several events occur within a predetermined period of time. Or, after having sent an interrogation request, sending another interrogation request may be blocked for a certain amount of time, to avoid unnecessary double requests.
For example, in case of an emergency, the remote server may receive emergency data indicating that emergency. Then, the remote server may send an interrogation request based on time data, e.g., considering opening times of respective health institutions and may provide the acquired medical device data to a health institution that is open at that time. Additionally or alternatively, in case of such emergency, the remote server may send an interrogation request based on location data, e.g., considering which suitable health institution is closest to the location where the emergency occurred and may provide the acquired medical device data to the respective health institution. The remote server may also consider the combination thereof by sending the medical device data to the closest health institution that is open at that time. The according choice of the respective health institution may for example also affect the interrogation request such that the parameters acquired by the medical device, e.g., an implant of the patient, and requested via the interrogation request may be adjusted to, e.g., the medical devices (e.g., medical imaging means) that may be available at the specific health institution.

In an example, the remote server may be configured to send the interrogation request to the at least one medical device only if at least (also) one of the following events occurs: (i) a current location of the patient is within a predetermined area of a location of the scheduled appointment, and (ii) a current time is within a predetermined range around the time of the scheduled appointment.

When the interrogation request is only sent based at least (also) on time and/or location data, it is ensured that the remote server, knowing at least the time(s) and/or location(s) of one or more appointments of the patient with one or more health professionals, may also know which health professional the medical device data need to be sent to. Therefore, no faulty and/or more targeted, respectively, data transmission may occur, and overall reliability of the remote server is increased.

The remote server may in an example further be configured to send the interrogation request to the medical device only if at least two of the events as described herein occur.

Two events may be associated with each other, e.g., location and time of a scheduled appointment, location and reason (emergency event) of a hospital visit, or two instances of emergency data from separate devices that may confirm that the patient indeed needs to be assisted by a health professional. The health professional may then, e.g., directly be provided with the medical device data for a remote assessment of the patient's condition.

For example, requiring the location and time to match location and time of an appointment avoids interrogating a patient's medical device who only happens to be at the location of the appointment (e.g. visiting a friend in the hospital) without the time of the appointment being close or a patient that has an appointment but does not show up at the location of the appointment.

In an example, the remote server may be configured to send the interrogation request to the medical device if a time between two events is below a first predetermined time interval (e.g., if the two events are roughly coincident) and/or refrain from sending the interrogation request to the medical device if the time between two events is not below the first predetermined time interval (e.g., if the two events are not coincident).

Two (associated) events may be considered coincident even though the remote server may not receive the respective data at the exact same time. For example, the patient may enter the doctor's office too early or too late for their appointment such that the time-related event and the location-related event occur at different times. A first predetermined time interval defining a maximum time difference (e.g., 5 minutes, 10 minutes, 15 minutes, 20 minutes, 25 minutes, 30 minutes, 1 hour or any other time) between two associated events mitigates/compensates for such issue, ensuring that interrogation requests are nevertheless sent (correctly), also in such situations where there are time differences between associated events. This may increase reliability und usability of the remote server and patient satisfaction. It optimizes the automated workflow of the remote server.

The remote server may for example be further configured to receive patient file data and send the interrogation request to the medical device at least in part based on the patient file data. Hence, for example the interrogation request may be tailored to the specific patient, e.g. preconditions, age, etc.

Patient file data may comprise one or more of, e.g., gender, age, weight, height, physiological parameters, information on medical conditions, treatment parameters, etc. These patient file data may be provided by the patient, e.g., via a remote device and/or by one or more health professionals, e.g., via a server running the patient data system. It may be especially useful to analyze emergency data by the remote server with reference to the patient file data to then decide whether the emergency data actually indicate an emergency event or not and accordingly whether an interrogation request is to be sent to the medical device or not. The interrogation request may then also be adjusted such that different parameters are requested according to the analysis of emergency data and patient file data. This may increase the reliability and flexibility to adjust the system to the patient's needs.

Generally, the comparison of patient file data with emergency data or any other data may also be implemented in any other component other than the remote server, e.g., in one or more remote devices and/or one or more medical devices.

The remote server may further be configured to send the interrogation request at most once within a second predetermined time interval. Additionally or alternatively, the remote server may in an exemplary embodiment be configured to send the interrogation request at one or more predetermined times and/or at a predetermined frequency.

This may prevent unwanted (repeat) interrogation of the medical device and improve automatization of remote monitoring of the patient's condition.

Defining such a minimum time interval between two subsequent interrogation requests may prevent that interrogation requests are sent unnecessarily often. For example, when a patient is waiting in the waiting room of a health institution, the current time may be within the predetermined range around the time of the scheduled appointment and their current location is within the predetermined area of the health institution. When both data are updated for example every minute, the interrogation request might be sent to the medical device every minute. It is clearly beneficial to prevent this by defining the second predetermined time interval. It may for example be set to 1 hour, 2 hours, 4 hours, 8 hours, 12 hours or 24 hours. It may also be set to any other time, according to the needs of the patient.

Further, additionally or alternatively, sending the interrogation request at one or more predetermined times, e.g., according to a monitoring plan scheduled by an assisting health professional and/or at a predetermined frequency, e.g., once a day, week, month, etc., the remote monitoring of the patient may be facilitated.

In a further example, the remote server may be configured to receive the medical device data from the medical device in response to the interrogation request, and preferably to provide the medical device data to a patient data system and/or other receiver(s). The (electronic) patient data system may for example be associated with a server at a health institution or be decentralized and the data may be automatically saved in an electronic patient file to be accessed by a health professional. It is also possible that the patient data of the medical device is additionally or alternatively provided to one or more remote devices.

The remote server may additionally or alternatively be configured to provide patient feedback to the patient when the medical device data are received. For example, a remote device, e.g., a mobile phone and/or a smart watch, located with the patient may provide patient feedback via a screen, speakers, or in form of haptic feedback. Thereby, the patient may be informed on the ongoing operation of the remote server, e.g. that and to which health professional his/her data were provided. Alternatively, the remote device(s) may be configured in an interactive way such that the patient may be asked to approve data transmission, may be asked to approve sending the interrogation request, may adapt the interrogation request and/or may decide which of the acquired medical device data may be transmitted to the remote server (and, preferably, ultimately to the patient data system), e.g., via a user interface of the remote device.

According to another aspect of the invention, a remote device for instructing a remote server for providing medical device data of a medical device of a patient is provided. The remote device is configured to receive at least one element of the following list of elements: (i) location data on a scheduled appointment of the patient, (ii) time data on a scheduled appointment of the patient, and/or (iii) emergency data on at least one emergency event associated with the patient. The remote device is further configured to send, at least in part based on the at least one received element, an interrogation instruction to the remote server, instructing the remote server to send an interrogation request to the medical device.

The interrogation request is of the form and is sent as described herein.

The location data, time data, emergency data, and interrogation instruction may be as described herein with reference to the remote server. Along with the interrogation instruction, (combinations of) the location data, time data, emergency data and/or parts thereof may be sent to the remote server, e.g., for further processing of the data, consistency checks, etc.

For example, the remote device may be configured to (automatically) compare the current time and location with the time and location of a scheduled appointment of the patient and send, based thereon, the interrogation instruction to the remote server, when both, current time and current location match time and location of that appointment.

Any functionality described herein in reference to the remote server and/or the patient data system may also be implemented in the remote device and vice versa.

According to a further aspect of the invention, a system is provided, wherein the system comprises at least two of the following: a remote server, a medical device, and a remote device (of the patient). All such components of the system may be configured as described herein. The system may also comprise a plurality of any such components.

Providing a system with mutually adapted components may further optimize the communication (speed, accuracy, etc.) of the conducted operations, enhancing the benefits of the automatization of the provision of medical device data of a medical device of a patient that the present invention enables.

The system may also comprise a patient data system. Such patient data system may in turn comprise patient file data and may be associated with any of the components, e.g., the remote server, the remote device, and/or the medical device. The patient data system may for example be a de-centralized system delocalized over multiple of the components, or it may run on a (separate and/or single) server.

Such system may comprise none, one and/or multiple of each of the listed components. For example, a system may comprise multiple remote devices, like a smartphone, a smartwatch, and/or a medical device-specific remote device (such as a dedicated control tool for, e.g., a neurostimulation device). In another example, such system may comprise multiple medical devices, e.g., an electrocardiograph, a pacemaker, and/or a ventricular assist device.

In any of the embodiments described herein, the medical device may be an at least partly implantable device.

The invention described herein is particularly advantageous for implants as they are often not visible from the first inspection of a patient. Thus, health professionals may often not even know if a patient has an implant, let alone the status of the according parameters measured by the implant indicating the potentially critical associated health status.

In an example, the one or more medical devices may be, e.g., electrocardiographs, pacemakers, ventricular assist devices, neurostimulation devices, etc.

According to another aspect of the invention, a method comprising the following steps is provided: Receiving, by a remote server, at least one element of the following list of elements: (i) location data on a scheduled appointment of the patient, (ii) time data on at least one scheduled appointment of the patient, (iii) emergency data on at least one emergency event associated with the patient,(iv) an interrogation instruction; and sending, by the remote server and at least in part based on the at least one element, an interrogation request to the medical device.

Operation of the remote server according to such method may provide some or all of the above-described advantages.

According to yet another aspect of the invention, a method comprising the following steps is provided: At least one element of the following list of elements is received by a remote device: (i) location data on a scheduled appointment of the patient, (ii) time data on a scheduled appointment of the patient, and/or (iii) emergency data on at least one emergency event associated with the patient. The remote device sends an interrogation instruction to the remote server, at least in part based on the at least one received element.

Operation of the remote device according to such method may provide some or all of the above-described advantages.

In a further aspect of the invention, a computer program is provided that comprises instructions which, when the computer program is executed, cause a remote server to carry out the method(s) as described above with reference to the remote server.

Such computer program may allow the remote server to realize the above-described advantages.

In another, further aspect of the invention, a computer program is provided that comprises instructions which, when the computer program is executed, cause a remote device to carry out the method(s) as described above with reference to the remote device.

Such computer program may allow the remote device to realize the above-described advantages.
- Fig. 1: Schematic representation of a system comprising a remote server, a patient data system, and an implanted medical device.
- Fig. 2: Schematic representation of a system comprising a remote server, a patient data system, an implanted medical device, and a remote device.
- Fig. 3: Schematic representation of another system comprising a remote server, a patient data system, an implanted medical device, and a remote device.
- Fig. 4: Schematic representation of a system comprising a remote server, a patient data system, an implanted medical device, a remote device, and a cloud-based location data database.

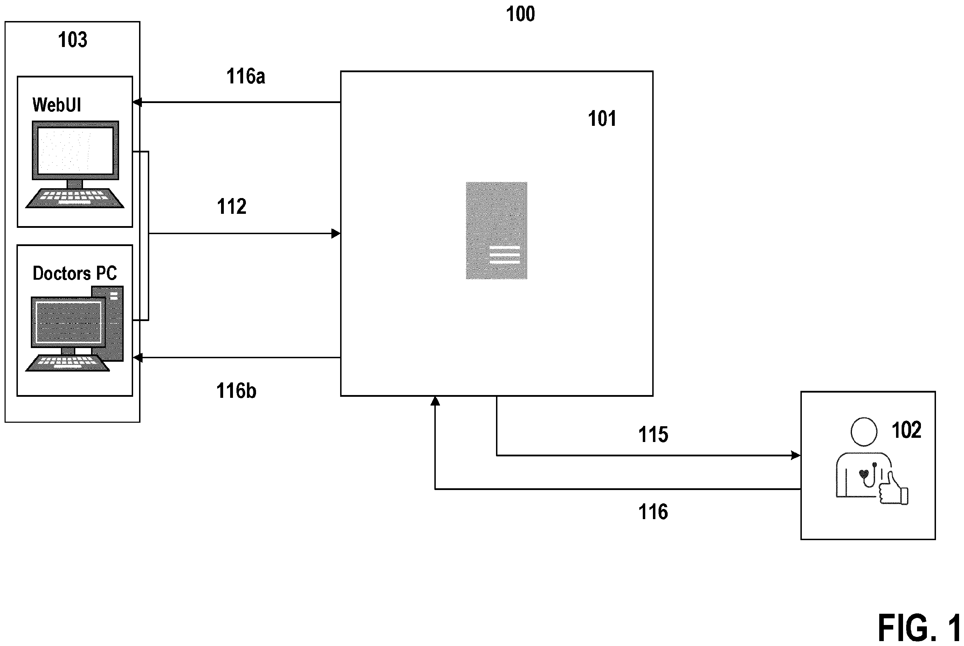

Figure 1 shows a schematic representation of an exemplary system 100 comprising a remote server 101, a patient data system 103, and an implanted medical device 102. The components 101, 102, 103 of the exemplary system 100 may for example be configured to communicate with each other in a wireless (e.g., via cellular radio (e.g., 5G, 4G, etc.), WiFi, infrared, satellite, microwave, Bluetooth) manner, via wires, and/or any other connection. Different communication pathways of the system 100 may be implemented via the same or different communication means.

In the exemplary communication pathways shown in Figure 1, interrogation on demand of the medical device 102, i.e., sending the interrogation request 115, is based on time data 112 in the following way: An appointment may be scheduled for a patient via the patient data system 103. The patient data system 103 may for example be part of the infrastructure of a hospital, doctor's office and/or any other health institution. The patient data system 103 may comprise for example one or more servers and/or computers, e.g., a doctors PC and/or an (online) web user interface (UI). The doctors PC may be coupled to the patient data system 103 for inputting and/or retrieving patient data. Analogously, the web UI may be accessible, e.g., also for patients. The patients and/or health professionals may for example schedule appointments, receive information on their appointments, their treatment, etc. via the web UI of the patient data system 103. The patient data system 103 may provide the remote server 101 with time data 112 on the scheduled appointment. At the time of the appointment or at a predetermined time before the scheduled appointment (e.g. as programmed via patient data system 113), the remote server 101 may send an interrogation request 115 to the implanted medical device 102. The interrogation request 115 may cause the medical device 102 to collect medical device data 116 from the patient and/or to retrieve medical device data 116 from a data log of the medical device 102. The medical device 102 may then provide medical device data 116 to the remote server 101, which may then transmit the medical device data 116a, 116b to the doctor's PC and the web UI of the patient data system 103, respectively. The medical device data 116a and 116b may be identical or different versions of the medical device data 116, respectively. For example, the medical device data 116a and 116b may be the same as the medical device data 116, a processed version, and/or part thereof. The medical device data 116a, 116b may comprise (parts of the) as-collected medical device data 116 on the health state of the patient and/or medical device data 116 from the data log of the medical device 102 and/or processed versions thereof. The remote server 101 and/or the medical device 102 may process the medical device data 116 before their transmission or directly transmit them to the patient data system 103 such that the health professional involved in the patient's appointment may have access to the medical device data 116a, 116b at the time of the appointment or already in advance for preparation of a check-up or medical intervention.

The whole interrogation on demand process may occur without the patient and/or the health professional noticing and/or being required to actively participate. The remote server 101 or any other component of the system 100 may simultaneously trigger such interrogation on demand for a plurality of patients and/or medical devices 102 of the same or different patients.

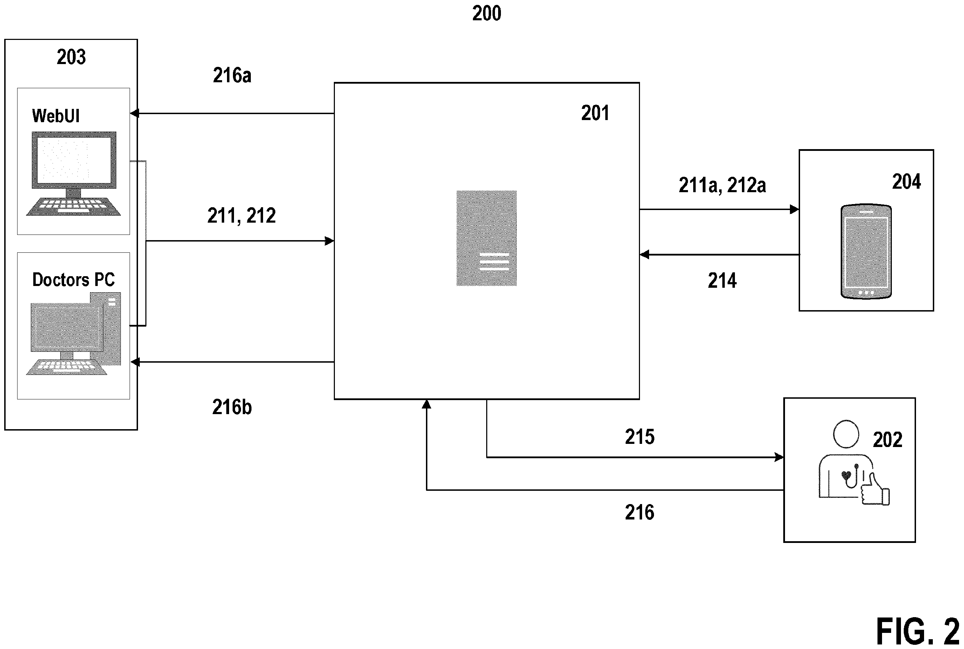

Figure 2 shows a schematic representation of an exemplary system 200 comprising a remote server 201, a patient data system 203, and an implanted medical device 202. The components 201, 202, 203 of the exemplary system 200 may for example be configured according to the exemplary embodiment of Figure 1. Additionally, the system 200 of Figure 1 comprises a remote device 204, in this example a mobile phone. The remote device 204 may, however, be any other suitable device, e.g., a smart watch, a tablet, a computer, a medical device-specific remote, etc.

The communication pathways of the individual components may be implemented similarly to the embodiment of Figure 1 as described herein. An appointment date, time, and location for a patient may be sent from the patient data system 203 to the remote server 201 in form of location data 211 and time data 212. The remote server 201 may transmit corresponding location data 211a and time data 212a to the remote device 204 which may in turn provide an interrogation instruction 214 to the remote server 201 at the time of the appointment, at a predetermined time before the appointment, and/or when the current location of the patient is within a predetermined area, e.g., an area associated with the location of a health institution. The location data 211a and time data 212a may be the same as location data 211 and time data 212, respectively, processed versions, and/or parts thereof. The system 200 may send interrogation request 215, receive medical device data 216 from the medical device 202, and send medical device data 216a, 216b to the patient data system 203 as described with reference to Figure 1. The medical device data 216a and 216b may be identical or different versions of the medical device data 216, respectively. The medical device data 216a and 216b may be the same as the medical device data 216, processed versions, and/or parts thereof.

The mobile phone, functioning as the remote device 204, of the exemplary system 200 may comprise a user interface informing the patient on the actions performed by the system 200 and/or provide possibilities for user input to adjust, pause, start, and/or stop the according operations of the system 200 and/or to provide additional time and/or location data 211 and/or to adjust them, e.g., when a scheduled appointment is cancelled or re-scheduled.

In some examples, only the location data 211a may be sent to remote device 204, and the remote device 204 may send the interrogation instruction 214, if the location data 211a indicate that the current location of the patient is within a predetermined area, e.g., an area associated with the location of a health institution. The remote server 201 may then for example send the interrogation request 215 when it has received the interrogation instruction 214 from the remote device 204 and/or the current time matches the time of the scheduled appointment and/or is a predetermined time before the scheduled appointment. Optionally, when both, receiving the interrogation instruction 214 and a time-match, are required by the remote server 201 for sending the interrogation request 215, there may be a condition such that the interrogation instruction 214 must be received by the remote server 201 during a predetermined time interval around the time of the scheduled appointment.

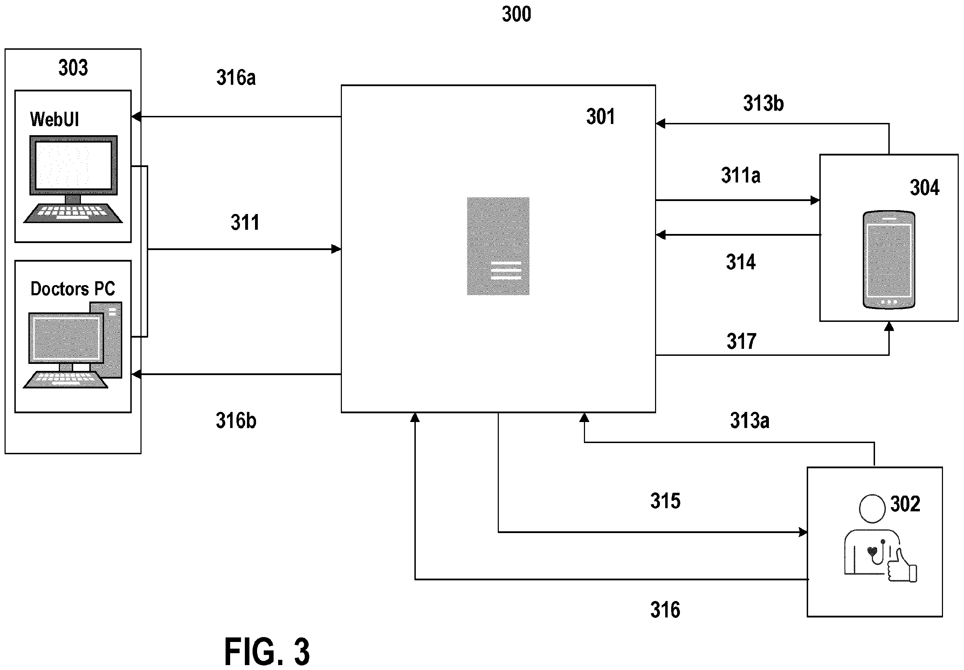

Figure 3 shows a schematic representation of an exemplary system 300 comprising a remote server 301, a patient data system 303, an implanted medical device 302, and a remote device 304. The components 301, 302, 303, 304 of the exemplary system 300 may for example be configured according to the exemplary systems 100 and/or 200 of Figures 1 and/or 2, respectively. The system 300 and its components 301, 302, 303, 304 may thus perform any function described in reference to Figures 1 and/or 2. The communication pathways of the individual components may be implemented analogously to the embodiment of Figure 1 and/or 2 as described herein. The remote server receives location data 311 and transmits location data 311a to the remote device 304. The location data 311a may be the same as location data 311, processed versions, and/or parts thereof. The remote server 301 may also receive time data on a scheduled appointment (not shown) which may be processed analogously to the location data 311. The system 300 may send an interrogation request 315, receive medical device data 316 from the medical device 302, and send the medical device data 316a, 316b to the patient data system 303 based on location data 311, 311a, time data (not shown), emergency data 313a, 313b, and/or an interrogation instruction 314. The medical device data 316a and 316b may be identical or different versions of the medical device data 316, respectively. The medical device data 316a and 316b may be the same as the medical device data 316, processed versions, and/or parts thereof.

Additionally, the remote device 304 of Figure 3 may comprise means configured to detect emergency events, e.g., falling of the patient, e.g., via accelerometers, changes in physiological parameters, etc. The remote device 304 may be a mobile phone and/or a smart watch, for example. When any such emergency event is detected by the remote device 304, emergency data 313b indicating an emergency event may be sent to the remote server 301.

Further, the medical device 302 may also be configured to monitor the health state of the patient according to at least one parameter. When the health state of the patient changes, e.g., the at least one parameter leaves a predetermined range, emergency data 313a indicating an emergency event may be sent to the remote server 301, analogously to the indication of an emergency by the remote device 304.

The remote server 301 may send an interrogation request 315 to the medical device 302 at least in part based on the emergency data 313a and/or the emergency data 313b. The remote device 304 and the medical device 302 may indicate the same emergency event or related events, and the remote server 301 may run a consistency check before sending an interrogation request 315 to the medical device 302. The consistency check may comprise matching the emergency data 313a and emergency data 313b to prevent sending unwanted information to health professionals. In further exemplary embodiments, a consistency check may additionally or alternatively be based on matching of any of the time data (not shown), location data 311, and/or the interrogation instruction 314 to prevent faulty interrogation requests 315 and accordingly to prevent sending unwanted information to health professionals. Optionally, the remote server 301 may send a confirmation 317 of the emergency, e.g., when the emergency data 313a and 313b are consistent in such that they both indicate an emergency event, to the remote device 304, e.g., to instruct the remote device 304 to send an emergency call to a health institution.

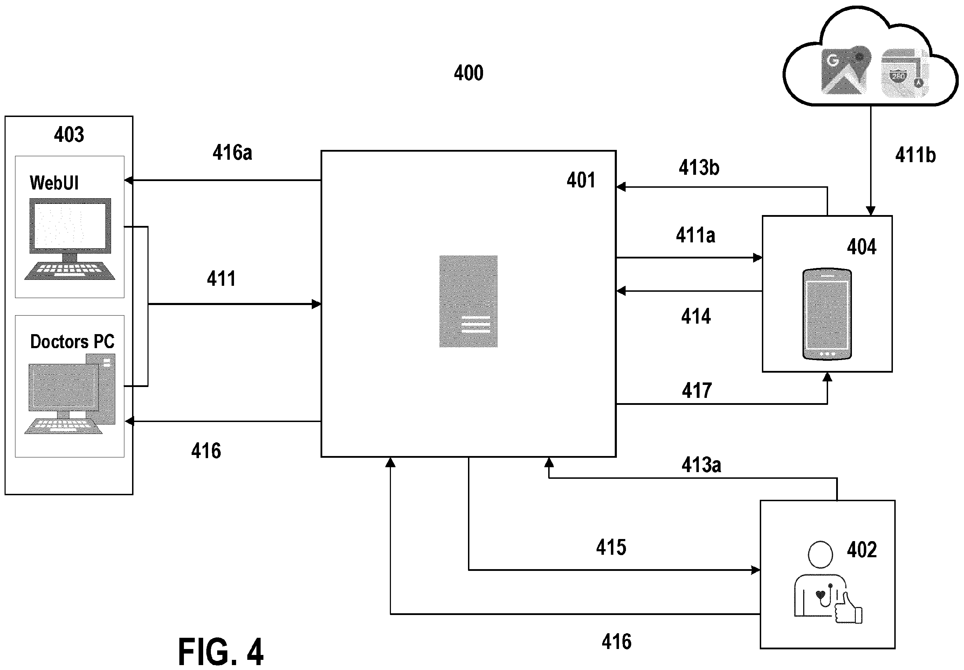

Figure 4 shows a schematic representation of an exemplary system 400 comprising a remote server 401, a patient data system 403, an implanted medical device 402, a remote device 404, and a location database 405. The location database 405 may be associated with the patient data system 403 or be completely independent, e.g., any online maps service. The components 401, 402, 403, and 404 of the exemplary system 400 may for example be configured according to the exemplary embodiments of Figures 1, 2, and/or 3. The system 400 and its components 401, 402, 403, and 404 may thus perform any function described with reference to Figures 1, 2, and/or 3. The communication pathways of the individual components may be implemented analogously to the embodiments of Figure 1, 2, and/or 3 as described herein. The system 400 may send an interrogation request 415, receive medical device data 416 from the medical device 402, and send the medical device data 416a, 416b to the patient data system 403 based on location data 411, 411a, 411b, time data (not shown), emergency data 413a, 413b, an emergency confirmation 417, and an interrogation instruction 414. The medical device data 416a and 416b may be identical or different versions of the medical device data 416, respectively. The medical device data 416a and 416b may be the same as the medical device data 416, processed versions, and/or parts thereof.

The location database 405 may be provided, for example, by any custom and/or openly available online maps service. Such services may be operably coupled to the remote device 404, e.g., a smartphone, which is typically compatible with such databases. The remote device 404 may download the location, e.g., hospitals and doctor's offices from the location database 405 to be comprised in the location data 411 and for matching with a current location of the remote device 404. The remote device may receive the location data 411a in addition to the location data 411b. The remote device 404 may compare the location data 411a on the current location with location data 411b on predetermined areas, e.g., associated with the locations of hospitals and only send the interrogation instruction 414 to the remote server 401 when the current location matches the predetermined area associated with, e.g., the location of a health institution. The location data 411a may be the same as location data 411, processed versions, and/or parts thereof.

Generally, any functionality described with reference to any component of the systems 100, 200, 300, 400 of Figures 1 through 4 may be implemented fully and/or partly in another component of the described systems 100, 200, 300, 400 and/or in an additional component, server, or device.

## Claims

1. A remote server (101, 201, 301, 401) for providing medical device data of a medical device (102, 202, 302, 402) of a patient, wherein the remote server is configured to: receive at least one element of the following list of elements:
(i) location data (211, 311, 411) on a scheduled appointment of the patient,
(ii) time data (112, 212) on a scheduled appointment of the patient,
(iii) emergency data (313, 413) on at least one emergency event associated with the patient, and
(iv) an interrogation instruction (214, 314, 414); and
send, at least in part based on the at least one element, an interrogation request (115, 215, 315, 415) to the medical device.

2. The remote server (101, 201, 301, 401) of claim 1, further configured to send the interrogation request (115, 215, 315, 415) to the medical device (102, 202, 302, 402) if at least one of the following events occurs:
(i) a current location of the patient is within a predetermined area of a location of the scheduled appointment,
(ii) a current time is within a predetermined range around a time of the scheduled appointment,
(iii) the emergency data (313, 413) on the at least one emergency event associated with the patient is received, and
(iv) the interrogation instruction (214, 314, 414) is received.

3. The remote server (101, 201, 301, 401) of claim 1 or 2, further configured to send the interrogation request (115, 215, 315, 415) to the at least one medical device (102, 202, 302, 402) only if at least one of the following events occurs:
(i) a current location of the patient is within a predetermined area of a location of the scheduled appointment, and
(ii) a current time is within a predetermined range around a time of the scheduled appointment.

4. The remote server (101, 201, 301, 401) of claim 2 or 3, further configured to send the interrogation request (115, 215, 315, 415) to the medical device (102, 202, 302, 402) only if at least two of the events occur.

5. The remote server (101, 201, 301, 401) of claim 4, further configured to:
send the interrogation request (115, 215, 315, 415) to the medical device (102, 202, 302, 402) if a time between the at least two events is below a first predetermined time interval, and/or
refrain from sending the interrogation request (115, 215, 315, 415) to the medical device (102, 202, 302, 402) if a time between the at least two events is not below the first predetermined time interval.

6. The remote server (101, 201, 301, 401) of any of the preceding claims, further configured to receive patient file data and send the interrogation request (115, 215, 315, 415) to the medical device (102, 202, 302, 402) at least in part based on the patient file data.

7. The remote server (101, 201, 301, 401) of any of the preceding claims, further configured to send the interrogation request (115, 215, 315, 415) at most once within a second predetermined time interval.

8. The remote server (101, 201, 301, 401) of any of the preceding claims, further configured to receive (116, 216, 316, 416) the medical device data from the medical device (102, 202, 302, 402) in response to the interrogation request (115, 215, 315, 415), and preferably to provide the medical device data to a patient data system.

9. A remote device (204, 304, 404) for instructing a remote server (101, 201, 301, 401) to provide medical device data of a medical device (102, 202, 302, 402) of a patient, wherein the remote device (204, 304, 404) is configured to:
receive at least one element of the following list of elements:
(i) location data (211, 311, 411) on scheduled appointment of the patient,
(ii) time data (112, 212) on a scheduled appointment of the patient, and
(iii) emergency data (313, 413) on at least one emergency event associated with the patient (313, 413); and
send, at least in part based on the at least one received element, an interrogation instruction (214, 314, 414) to the remote server (101, 201, 301, 401).

10. A system comprising at least two of the following:
a remote server (101, 201, 301, 401) according to any of claims 1 to 8;
a medical device (102, 202, 302, 402); and
a remote device (204, 304, 404) according to claim 9.

11. The remote server (101, 201, 301, 401) of any of claims 1 to 8, the remote device (204, 304, 404) of claim 9, or the system of claim 10, wherein the medical device (102, 202, 302, 402) is an at least partly implantable device.

12. A method comprising the following steps:
receiving, by a remote server (101, 201, 301, 401), at least one element of the following list of elements:
(i) location data (211, 311, 411) on a scheduled appointment of the patient,
(ii) time data (112, 212) on a scheduled appointment of the patient,
(iii) emergency data (313, 413) on at least one emergency event associated with the patient, and
(iv) an interrogation instruction (214, 314, 414); and
sending, by the remote server (101, 201, 301, 401) and at least in part based on the at least one element, an interrogation request (115, 215, 315, 415) to the medical device (102, 202, 302, 402).

13. A method comprising the following steps:
receiving, by a remote device (204, 304, 404), at least one element of the following list of elements:
(i) location data (211, 311, 411) on a scheduled appointment of the patient,
(ii) time data (112, 212) on a scheduled appointment of the patient, and
(iii) emergency data (313, 413) on at least one emergency event associated with the patient; and
sending, by the remote device (204, 304, 404) and at least in part based on the at least one received element, an interrogation instruction (214, 314, 414) to the remote server (101, 201, 301, 401).

14. A computer program comprising instructions which, when the computer program is executed, cause a remote server (101, 201, 301, 401) to carry out the method of claim 12.

15. A computer program comprising instructions which, when the computer program is executed, cause a remote device (204, 304, 404) to carry out the method of claim 13.
